# EUROPÄISCHE PATENTSCHRIFT

(11) **EP 3 797 804 B1**
(45) Veröffentlichungstag und Bekanntmachung des Hinweises auf die Patenterteilung: **28.01.2026**
(21) Anmeldenummer: 20197629.7
(22) Anmeldetag: 23.09.2020
(51) Int. Cl.: A61M 1/34

(54) **BLUTBEHANDLUNGSVORRICHTUNG MIT AUTOMATISCHER SUBSTITUTIONSVOLUMENKOMPENSATION**
BLOOD TREATMENT DEVICE WITH AUTOMATIC SUBSTITUTION VOLUME COMPENSATION
DISPOSITIF DE TRAITEMENT DU SANG À COMPENSATION AUTOMATIQUE DU VOLUME DE SUBSTITUTION

(30) Priorität: 26.09.2019 DE 102019126048
(43) Veröffentlichungstag der Anmeldung: 31.03.2021
(73) Patentinhaber: B. Braun Avitum AG, 34212 Melsungen (DE)
(72) Erfinder: GOLARITS, István, 1073 Budapest (HU); PÓZNA, Peter, 1158 Budapest (HU); TÉNYI, Botond, 1037 Budapest (HU)
(74) Vertreter: Winter, Brandl - Partnerschaft mbB

(56) Entgegenhaltungen:
- EP-A1- 0 321 754
- EP-A1- 0 373 455
- EP-A1- 3 015 123
- US-A1- 2002 150 476

## Beschreibung

Die vorliegende Offenbarung betrifft eine Blutbehandlungsvorrichtung, insbesondere Dialysemaschine, zur Verwendung in (kontinuierlichen) Blutbehandlungs-/ Dialysetherapien, insbesondere Nierenersatztherapien, mit: einem extrakorporalen Blutkreislauf, einem Dialysator und einem Dialysierflüssigkeitskreislauf, wobei der extrakorporale Blutkreislauf und der Dialysierflüssigkeitskreislauf über eine in dem Dialysator vorgesehene Membran, über welche Blut (unter Verwendung einer Dialysierflüssigkeitslösung) gefiltert werden kann, voneinander getrennt sind; und zumindest einer Substitutionslösungspumpe, welche eingerichtet ist, eine Substitutionslösung dem extrakorporalen Blutkreislauf vor und/oder nach dem Dialysator zuzuführen.

### Stand der Technik

Aus dem Stand der Technik sind bereits Blutbehandlungsvorrichtungen bekannt. Zum Beispiel offenbart die EP 0 321 754 A1 eine Blutbehandlungsvorrichtung mit einem Filter, der durch eine Membran in zwei Kammern geteilt ist. Durch eine Kammer des Filters ist ein extrakorporaler Blutkreislauf gelegt. Die andere Kammer des Filters ist mit einer Ultrafiltrationseinheit verbunden, welche eingerichtet ist, aus der anderen Kammer mittels einer Ultrafiltratpumpe Ultrafiltrat zu entnehmen. Die Blutbehandlungsvorrichtung weist eine Substitutionseinheit auf, welche eingerichtet ist, dem Blutkreislauf mittels einer Substituatpumpe eine Substitutionsflüssigkeit zuzuführen. Weiterhin weist die Blutbehandlungsvorrichtung eine Waage auf, die die entnommene Ultrafiltratmenge und die zugeführte Substitutionsflüssigkeitsmenge gegeneinander bilanziert, indem der Ultrafiltratbehälter und der Substitutionsflüssigkeitsbehälter mit ihrem jeweiligen Inhalt gewogen werden. Darüber hinaus enthält die Blutbehandlungsvorrichtung eine Steuereinheit zur Steuerung der Ultrafiltratpumpe und der Substituatpumpe.

Auch offenbart ein anderes Dokument, die EP 0 829 265 B1 eine Blutbehandlungsvorrichtung, welche eine Schnittstelle für ein Einwegschlauchset, eine Vielzahl von Pumpen wie eine Blutpumpe, eine Spritzenpumpe, eine Ultrafiltratpumpe und eine Substitutionspumpe, Wägezellen zum Messen des Gewichts von Beuteln, welche für die Blutbehandlung erforderliche Fluide enthalten, eine Benutzeroberfläche umfassend ein Display mit Touchscreen und eine Steuereinheit zum Steuern der Prozesse der Blutbehandlungsvorrichtung aufweist.

Die EP 3 015 123 A1 offenbart eine Blutbehandlungsvorrichtung zur Verwendung in Blutbehandlungstherapien, mit einem extrakorporalen Blutkreislauf, einem Dialysator und einem Dialysierflüssigkeitskreislauf, wobei der extrakorporale Blutkreislauf und der Dialysierflüssigkeitskreislauf über eine in dem Dialysator vorgesehene Membran, über welche Blut gefiltert werden kann, voneinander getrennt sind. Die Blutbehandlungsvorrichtung weist zumindest eine Substitutionslösungspumpe auf, welche eingerichtet ist, eine Substitutionslösung dem extrakorporalen Blutkreislauf vor und/oder nach dem Dialysator zuzuführen. In der EP 3 015 123 A1 wird offenbarungsgemäß eine Abweichung zwischen einem vorgegebenen Sollvolumen und einem Istvolumen einer zugeführten Substitutionslösung berechnet. Die Abweichung des Substitutionsvolumens wird zu einem Substitutionskorrekturwert oder -betrag verarbeitet, der dann als Prozentsatz des eingestellten Substitutionsflusses oder in einer anderen geeigneten Form ausgegeben werden kann. Ein geregelter Substitutionsfluss wird um den zuvor ermittelten Prozentsatz korrigiert. Die EP 3 015 123 A1 stellt eine komplexe Regelung ("feedback control") zur Kompensation der Abweichung des Substitutionsvolumens bereit.

US 2002/150476 A1 offenbart eine Steuerung mit verschiedenen Modi für eine Fluidpumpe für eine Blutbehandlungsvorrichtung. In einem Back Off Response Mode kann ein Parameter, wie die Flussrate der Pumpe, gesteuert werden, indem die Geschwindigkeit der Pumpe solange schrittweise um einen festen Prozentsatz reduziert wird, bis die Flussrate nicht mehr oberhalb eines Sollwerts liegt.

Weiterer Stand der Technik findet sich in der EP 0 373 455 A1, der CA 2 580 848 A1, der US 5,470,483 A, der WO 94/11093 A1, der DE 33 13 421 A1, der WO 92/00768 A1, der WO 2018/017623 A1, und der US 9,089,639 B2.

Während einer Dialysebehandlung können grundsätzlich Ereignisse eintreten, die dazu beitragen, dass ein angestrebtes ideales/ optimales, durch einen Anwender eingestelltes Soll-Substitutionsfluidvolumen/ Substitutionslösungsvolumen nicht erreicht werden kann. Beispielsweise kann dies beim Hochfahren/ Starten/ erneutem Starten der Substitutionslösungspumpe/ der Pumpenflussrate zu Beginn einer Therapie der Fall sein. Darüber hinaus können Fehler in der Zuführung der Substitutionslösung auftreten, beispielsweise, wenn ein die Substitutionslösung enthaltender Beutel nicht korrekt an einen Schlauch angeschlossen ist, welcher die Substitutionslösung dem extrakorporalen Blutkreislauf zuführt.

Der Stand der Technik hat grundsätzlich den Nachteil, dass Abweichungen (das heißt Unterschiede/ Rückstände) zwischen einem (vorbestimmten) idealen/ optimalen, durch einen Anwender eingestellten Sollvolumen und einem tatsächlich/ wirklich gesteuerten Volumen der zugeführten Substitutionslösung nicht in einfacher Weise automatisch kompensiert werden können.

### Kurzbeschreibung der Offenbarung

Es ist also Aufgabe der vorliegenden Offenbarung, die Nachteile aus dem Stand der Technik zu vermeiden oder wenigstens zu mildern. Insbesondere soll die Blutbehandlungsvorrichtung so eingerichtet sein, dass sie Abweichungen/ Unterschiede zwischen einem idealen/ optimalen Sollvolumen und einem tatsächlich gesteuerten Volumen der zugeführten Substitutionslösung bzw. Rückstände des tatsächlich gesteuerten Volumens im Hinblick auf das ideale Sollvolumen über den Verlauf einer Therapie gesehen allmählich kompensiert, so dass letztendlich das ideale (erwünschte) Sollvolumen (wieder) erreicht wird.

Diese Aufgabe wird bei einer gattungsgemäßen Blutbehandlungsvorrichtung dadurch gelöst, dass sie eine Steuereinheit aufweist, welche eingerichtet ist, einen Rückstand zwischen einem (vorbestimmten) idealen/ optimalen, durch einen Anwender eingestellten Sollvolumen und einem tatsächlich/ wirklich gesteuerten Volumen der zugeführten Substitutionslösung zu berechnen, und eine gesteuerte (Durch-) Flussrate/ Förderleistung/ Fördermenge/ einen gesteuerten Volumenstrom der Substitutionslösungspumpe temporär unter entsprechender Ansteuerung derselben um einen vorbestimmten festen Prozentsatz, welcher kleiner oder gleich 5 % ist, (im Vergleich zu einer voreingestellten/ ursprünglichen bzw. normalen/ erwünschten/ eigentlich erforderlichen Flussrate der Substitutionslösungspumpe) zu erhöhen, und zwar so lange, bis die Abweichung zwischen dem tatsächlich gesteuerten Volumen und dem idealen Sollvolumen nicht mehr vorliegt, also das tatsächlich gesteuerte Volumen dem idealen Sollvolumen entspricht.

In anderen Worten berechnet die Steuereinheit der vorliegenden Offenbarung eine Abweichung zwischen Volumina, und zwar zwischen einem Sollvolumen der Substitutionslösung, welches dem extrakorporalen Blutkreislauf zugeführt werden soll, und einem tatsächlich zugeführten/ gesteuerten Volumen/ einem Ist-Volumen/ einer tatsächlich zugeführten/ gelieferten Menge. Insbesondere wird ein Rückstand/ ein Nachholbedarf des tatsächlichen Volumens im Vergleich zu dem Sollvolumen berechnet. Liegt ein Rückstand vor, wird die Flussrate der Substitutionspumpe/ die Substitutionslösungsflussrate temporär, also vorübergehend für einen bestimmten Zeitraum, erhöht. Kern der Offenbarung ist es, dass die prozentuale Erhöhung der Flussrate der Substitutionspumpe maximal 5% ist. Es wird von der Steuereinheit dementsprechend eine neue, erhöhte Flussrate eingestellt. Sobald keine Differenz zwischen dem tatsächlich gesteuerten Volumen und dem Sollvolumen/ kein Rückstand mehr vorliegt, wird wieder die ursprüngliche/ normale/ voreingestellte/ eigentlich erforderliche/ erwünschte Flussrate der Substitutionslösungspumpe eingestellt. Abweichungen zwischen dem idealen Sollvolumen und dem tatsächlich gesteuerten Volumen der zugeführten Substitutionslösung können somit offenbarungsgemäß in einfacher Weise automatisch kompensiert werden.

In anderen Worten ist die Steuereinheit gemäß der vorliegenden Offenbarung eingerichtet, den Rückstand zwischen dem idealen Sollvolumen und dem tatsächlich gesteuerten Volumen zu kompensieren, indem die gesteuerte Flussrate der zumindest einen Substitutionslösungspumpe temporär um den vorbestimmten, festen Prozentsatz erhöht wird.

Vorteilhafte Ausführungsformen sind in den Unteransprüchen beansprucht und werden nachfolgend erläutert.

Es ist vorteilhaft, wenn der vorbestimmte, feste Prozentsatz, um welchen der Volumenstrom der Substitutionslösung erhöht wird, zumindest 1% und höchstens 5% ist. Wenn der Prozentsatz zwischen 1% und 5% ist, wird die Abweichung bzw. der Rückstand zeitnah, jedoch auch nicht zu schnell beseitigt, so dass die Steuereinheit rechtzeitig reagieren kann, wenn das tatsächlich gesteuerte Volumen dem idealen Sollvolumen entspricht, und die Flussrate der Substitutionslösungspumpe wieder auf den (eigentlich erwünschten) Ausgangswert zurücksetzen kann. Es wird somit bevorzugt ausgeschlossen, dass das tatsächlich gesteuerte Volumen während einer Erhöhung der Flussrate größer als das Sollvolumen wird. Darüber hinaus hat sich herausgestellt, dass, wenn der Prozentsatz größer als 1% ist, der tatsächliche Volumenstrom/ die tatsächliche Flussrate im Normalfall groß genug wird, um die oben angesprochenen Ereignisse, die dazu beitragen, dass das angestrebte Substitutionsfluidvolumen/ Substitutionslösungsvolumen nicht erreicht wird, zu kompensieren.

Bevorzugt wird der vorbestimmte, feste Prozentsatz von der Steuereinheit in Abhängigkeit von dem fehlenden Volumen eingestellt, so dass der vorbestimmte, feste Prozentsatz höher eingestellt wird, wenn die Abweichung zwischen dem tatsächlich gesteuerten Volumen und dem idealen Sollvolumen groß ist als wenn die Abweichung zwischen dem tatsächlich gesteuerten Volumen und dem idealen Sollvolumen klein ist. Beispielsweise wird der vorbestimmte, feste Prozentsatz auf 1% eingestellt, wenn die Abweichung zwischen dem tatsächlich gesteuerten Volumen und dem idealen Sollvolumen klein ist, und wird der vorbestimmte, feste Prozentsatz auf 5% eingestellt, wenn die Abweichung zwischen dem tatsächlich gesteuerten Volumen und dem idealen Sollvolumen groß ist. Es ist jedoch auch jede prozentuale Erhöhung zwischen 1% und 5% denkbar.

Weiterhin ist es von Vorteil, wenn bei der Berechnung der Abweichung zwischen dem tatsächlichen Volumen und dem Sollvolumen durch einen Alarm ausgelöste Therapiestoppzeiten berücksichtigt werden. Wenn ein Alarm ausgelöst wird, wird grundsätzlich eine Therapie gestoppt. Es wird demnach auch keine Substitutionslösung dem extrakorporalen Blutkreislauf zugeführt. Während des Alarms/ Therapiestopps muss demnach kein Fluidvolumen kompensiert werden und die Steuereinheit berechnet demnach eine zugeführte/ gelieferte Menge während des Alarms/ Therapiestopps nicht mit ein.

Es ist von Vorteil, wenn die Steuereinheit eingerichtet ist, einen Alarm auszugeben, wenn sie feststellt, dass selbst bei einer Erhöhung der Flussrate der Substitutionslösungspumpe um 5% die Abweichung zwischen dem tatsächlichen Volumen und dem Sollvolumen nicht kompensiert werden kann.

In vorteilhafter Weise ist die Steuereinheit eingerichtet, die Flussrate der Substitutionslösungspumpe nur dann zu erhöhen, wenn dies nicht andere Restriktionen/ Bedingungen verbieten.

Bevorzugt ist die Steuereinheit eingerichtet, die Differenz bzw. den Rückstand zwischen dem idealen Sollvolumen und dem tatsächlich gesteuerten Volumen unter Verwendung des Verlaufs der (von der Steuereinheit eingestellten) Flussrate der zumindest einen Substitutionslösungspumpe zu berechnen.

Es ist dabei zweckmäßig, wenn die Steuereinheit eingerichtet ist, die Flussrate bzw. den Volumenstrom der zumindest einen Substitutionslösungspumpe einzustellen.

Bevorzugt erhöht sich beim (erneuten) Starten der zumindest einen Substitutionslösungspumpe die Flussrate/ der Volumenstrom langsam/ kontinuierlich/ linear, so dass eine erwünschte Flussrate/ ein erwünschter Volumenstrom erst nach einer vorbestimmten kurzen Zeitspanne erreicht wird.

Insbesondere steigt der Volumenstrom/ die Flussrate beim Starten/ erneuten Starten linear von null bis zu der erwünschten/ gewünschten Flussrate (Volumenstrom) an.

Bevorzugt ist die Steuereinheit eingerichtet, die Flussrate nach einem Erreichen der erwünschten/ gewünschten Flussrate/ des erwünschten Volumenstroms temporär um den vorbestimmten festen Prozentsatz zu erhöhen, um die Differenz/ den Rückstand zwischen dem tatsächlich gesteuerten Volumen und dem idealen Sollvolumen, welcher von der langsamen/ kontinuierlichen Erhöhung der Flussrate beim Starten/ erneuten Starten resultiert, langsam/ kontinuierlich zu verringern, und zwar so lange, bis die Differenz bzw. der Rückstand zwischen dem tatsächlich gesteuerten Volumen und dem idealen Sollvolumen nicht mehr vorliegt, also das tatsächlich gesteuerte Volumen dem idealen Sollvolumen entspricht.

In vorteilhafter Weise ist die Steuereinheit ferner eingerichtet, wenn die gesteuerte Flussrate der Substitutionslösungspumpe temporär reduziert werden muss (beispielsweise aufgrund eines temporären Blockierens des Dialysators), den daraus resultierenden Rückstand bzw. die daraus resultierende Differenz zwischen dem idealen Sollvolumen und dem tatsächlich gesteuerten Volumen (im Nachhinein) wieder zu verringern bzw. auszugleichen, indem die gesteuerte Flussrate der Substitutionslösungspumpe temporär unter entsprechender Ansteuerung derselben um den vorbestimmten festen Prozentsatz erhöht wird, und zwar so lange, bis die Differenz bzw. der Rückstand zwischen dem tatsächlich gesteuerten Volumen und dem idealen Sollvolumen nicht mehr vorliegt, also das tatsächlich gesteuerte Volumen dem idealen Sollvolumen entspricht.

Die Steuereinheit kann grundsätzlich auch eingerichtet sein, in dem Fall, dass ein tatsächlich gesteuertes Volumen der zugeführten Substitutionslösung größer als das ideale, durch den Anwender eingestellte Sollvolumen ist, die gesteuerte Flussrate der Substitutionslösungspumpe temporär um einen/ den vorbestimmten festen Prozentsatz zu verringern, und zwar solange, bis keine Abweichung mehr zwischen dem tatsächlich gesteuerten Volumen und dem idealen Sollvolumen mehr vorliegt, also das tatsächlich gesteuerte Volumen dem idealen Sollvolumen entspricht.

Bevorzugt weist die Blutbehandlungsvorrichtung eine Wägevorrichtung, insbesondere Wägezelle, zum Messen des Gewichts eines Beutels, insbesondere Einwegbeutels, welcher die Substitutionslösung enthält, auf.

Es ist zweckmäßig, wenn der extrakorporale Blutkreislauf und der Dialysierflüssigkeitskreislauf als Einwegschläuche ausgebildet sind, welche an einer an der Dialysemaschine vorgesehenen Schnittstelle aufgenommen sind.

Bevorzugt umfasst die Vielzahl von Pumpen neben der Substitutionslösungspumpe zumindest eine Blutpumpe, eine Spritzenpumpe und eine Ultrafiltratpumpe.

Die Blutbehandlungsvorrichtung weist ferner vorzugsweise ein Bar-Code-Lesegerät auf, welches eingerichtet ist, auf Einwegartikel wie etwa Einwegschläuche oder auf deren Umverpackung aufgebrachte Bar-Codes zu lesen.

Weiterhin umfasst die Blutbehandlungsvorrichtung bevorzugt eine Benutzeroberfläche umfassend ein Display mit Touch-Screen.

Die Blutbehandlungsvorrichtung ist bevorzugt zur drahtgebundenen Kommunikation eingerichtet.

Die Steuereinheit der Blutbehandlungsvorrichtung ist vorzugsweise als zumindest ein Prozessor, vorzugsweise mehrere Prozessoren, ausgebildet.

Mit anderen Worten betrifft die Offenbarung eine Dialysemaschine. Die Dialysemaschine enthält ein Bar-Code-Lesegerät. Weiterhin enthält die Dialysemaschine eine Benutzeroberfläche bzw. ein Display mit Touch-Screen. Die Dialysemaschine weist ferner eine Schnittstelle/ Interface für ein Einwegschlauchset auf, welches eine Blutseite und eine Dialysierflüssigkeitsseite enthält, die voneinander durch eine (semi-) permeable/ durchlässige Membran getrennt sind, um Blut (unter Verwendung einer Dialysierflüssigkeitslösung/ Dialyselösung) zu filtern. Eine Substitutionslösung/ Ersatzlösung wird der Blutseite vor/nach einem Dialysator zugeführt. Die Dialysemaschine weist eine Blutpumpe, eine Spritzenpumpe, eine Ultrafiltratpumpe, eine Substitutionslösungspumpe etc. auf. Die Dialysemaschine ist zur drahtgebundenen Kommunikation eingerichtet/ weist verdrahtete bzw. drahtgebundene Kommunikationseinrichtungen auf. Die Dialysemaschine zeichnet sich durch eine Software aus, welche besonders für eine Verwendung in kontinuierlichen Dialysetherapien, beispielsweise Nierenersatztherapien geeignet ist. Die Software läuft dabei auf einer Vielzahl von Prozessoren innerhalb der Dialysemaschine. Weiterhin weist die Dialysemaschine eine Energieverwaltungseinrichtung (integrierter Schaltkreis) auf. Die Dialysemaschine enthält darüber hinaus Wägevorrichtungen, insbesondere Wägezellen, welche das Gewicht von Einwegbeuteln messen, welche die für die Dialysetherapie erforderlichen Fluide (z.B. Dialysierflüssigkeitslösung, Substitutionslösung) enthalten.

Das vorliegende System bzw. die Dialysemaschine ist so konzipiert, dass Rückstände zwischen dem tatsächlichen Volumen und dem Sollvolumen der Substitutionslösung im Laufe der Therapie kompensiert werden, so dass letztendlich das Sollvolumen erreicht wird. Wenn das System eine Abweichung zwischen Soll- und Istvolumen feststellt, wird die Substitutionsfluidflussrate vorübergehend um 1 % bis 5 % erhöht (in Abhängigkeit von dem fehlenden Volumen). Wenn der Rückstand/ die Abweichung beseitigt ist, wird diese Funktion abgeschaltet.

### Kurzbeschreibung der Figuren

Die Offenbarung wird nachfolgend mit Hilfe von Figuren weiter erläutert. Es zeigen:
- Fig. 1: eine schematische Ansicht einer Blutbehandlungsvorrichtung gemäß der vorliegenden Offenbarung;
- Fig. 2: ein Flussdiagramm, welches die offenbarungsgemäße, in der Steuereinheit ablaufende automatische Kompensation eines Volumens der Substitutionslösung veranschaulicht; und
- Fig. 3: ein Diagramm, in welchem ein zeitlicher Verlauf einer Substitutionslösungsflussrate gemäß der vorliegenden Offenbarung dargestellt ist.

### Figurenbeschreibung

Die Figuren sind lediglich schematischer Natur und dienen ausschließlich dem Verständnis der vorliegenden Offenbarung. Gleiche Elemente sind dabei mit denselben Bezugszeichen versehen.

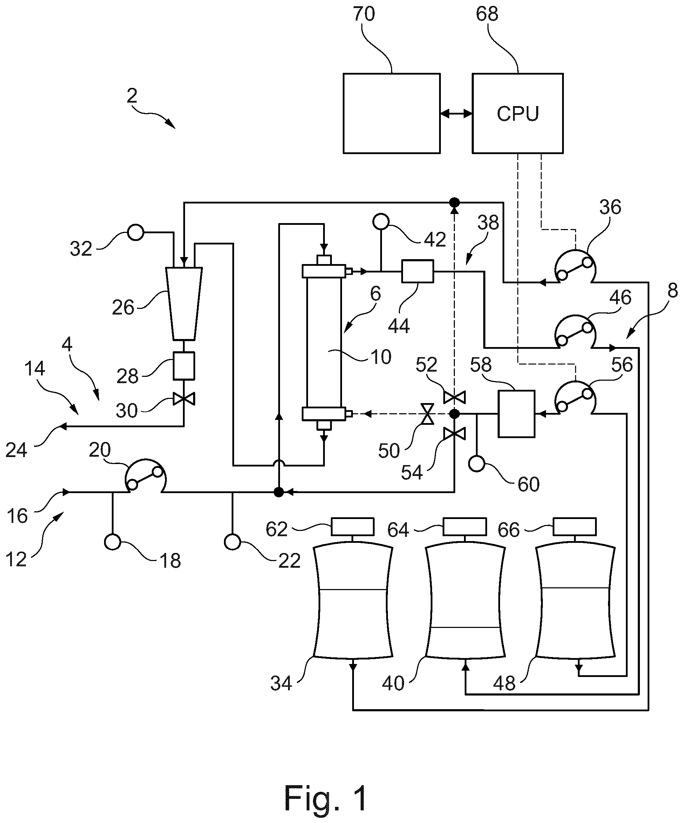

Fig. 1 zeigt eine schematische Ansicht einer extrakorporalen Blutbehandlungsvorrichtung (Dialysemaschine) 2. Die Blutbehandlungsvorrichtung 2 ist grundsätzlich eingerichtet, sowohl in kontinuierlichen als auch in intermittierenden Blutbehandlungstherapien, insbesondere Nierenersatztherapien, verwendet zu werden. Die Blutbehandlungsvorrichtung 2 ist insbesondere als eine Akutdialysemaschine bzw. als ein Akut-Dialysegerät ausgebildet und ist somit im Wesentlichen zur Verwendung auf Intensivstationen bei überwiegend instabilen Patienten vorbereitet. Mit der Blutbehandlungsvorrichtung 2 der vorliegenden Offenbarung können grundsätzlich eine Vielzahl von verschiedenen Blutbehandlungstherapien (z.B. langsame kontinuierliche Ultrafiltration (SCUF), kontinuierliche venös-venöse Hämofiltration (CVVH), kontinuierliche venös-venöse Hämodialyse (CVVHD), kontinuierliche venös-venöse Hämodiafiltration (CVVHDF), therapeutischer Plasmaaustauch (TPE), etc.), Dilutionsmodi (z.B. Prädilution, Postdilution, Prä- und Postdilution), sowie Antikoagulationsarten (z.B. keine, Heparin, Citrat, etc.) durchgeführt werden.

Die Blutbehandlungsvorrichtung 2 weist grundsätzlich einen extrakorporalen Kreislauf 4, einen Dialysator (Hämofilter) 6 und einen Dialysierflüssigkeitskreislauf 8 auf. Der extrakorporale Kreislauf 4 und der Dialysierflüssigkeitskreislauf 8 sind über eine in dem Dialysator 6 vorgesehene Membran 10, über welche Blut unter Verwendung einer Dialysierflüssigkeitslösung oder ohne Verwendung einer Dialysierflüssigkeitslösung gefiltert werden kann, voneinander getrennt.

Der extrakorporale Kreislauf 4 umfasst einen arteriellen Abschnitt 12 und einen venösen Abschnitt 14. Dabei ist grundsätzlich vorgesehen, dass der arterielle Abschnitt 12, insbesondere ein Ende desselben, an eine Arterie eines Patienten, insbesondere eines Intensivpatienten, angeschlossen bzw. mit derselben verbunden werden soll. Weiterhin ist vorgesehen, dass der venöse Abschnitt 14, insbesondere ein Ende desselben, an eine Vene eines Patienten, insbesondere eines Intensivpatienten, angeschlossen bzw. mit derselben verbunden werden soll.

Der arterielle Abschnitt 12 weist ausgehend von einem arteriellen Ende 16 in einer Blut-Strömungsrichtung hin zu dem Dialysator 6 einen arteriellen Drucksensor 18, eine (arterielle) Blutpumpe 20 und einen Dialysatoreingangsdrucksensor 22 auf. Der venöse Abschnitt 14 weist ausgehend von dem Dialysator 6 in einer Blut-Strömungsrichtung hin zu einem venösen Ende 24 eine venöse Expansionskammer bzw. Luftfalle 26, einen Sicherheitsluftdetektor 28 und ein Sicherheitsventil 30 auf. An/ hinter der venösen Expansionskammer 26 kann ein venöser Druck über einen venösen Drucksensor 32 gemessen werden.

Wie aus Fig. 1 hervorgeht, ist die venöse Expansionskammer 26 mit einem Substitutionslösungsbeutel/ -behälter 34 verbunden. Eine Substitutionslösungspumpe 36 ist dabei vorgesehen und eingerichtet, eine Substitutionslösung aus dem Substitutionslösungsbeutel 34 in den extrakorporalen Blutkreislauf 4, insbesondere in den venösen Abschnitt 14 desselben (in die venöse Expansionskammer 26) zu pumpen.

Der Dialysierflüssigkeitskreislauf 8 weist zumindest einen Abfluss 38 für Ultrafiltrat/ verbrauchte Dialysierflüssigkeit (Dialysat)/ eine sonstige Flüssigkeit auf. Grundsätzlich kann das Ultrafiltrat/ Dialysat/ die sonstige Flüssigkeit über den Abfluss 38 von dem Dialysator 6 hin zu einem Sammelbeutel/ -behälter 40 für Ultrafiltrat/ Dialysat/ etc. fließen. In dem Abfluss 38 sind ausgehend von dem Dialysator 6 in einer Strömungsrichtung hin zu dem Sammelbeutel 40 ein Ultrafiltratdrucksensor 42, ein Blutleckdetektor 44 und eine Ultrafiltratpumpe 46 angeordnet bzw. vorgesehen.

Wie aus Fig. 1 weiter hervorgeht, ist neben dem Substitutionslösungsbeutel 34 und dem Sammelbeutel 40 noch ein weiterer Beutel/ Behälter 48 vorgesehen. Der Beutel 48 kann in Abhängigkeit von der gewünschten durchzuführenden Blutbehandlungstherapie beispielsweise eine Substitutionslösung /-flüssigkeit oder eine Dialysierflüssigkeit enthalten.

Wenn beispielsweise mit der extrakorporalen Blutbehandlungsvorrichtung 2 eine Hämodialyse-/ eine Hämodiafiltrationsbehandlung etc. durchgeführt werden soll, also eine Blutbehandlungstherapie, in welcher Dialysierflüssigkeit durch den Dialysator 6 strömt und somit ein Stofftransport von dem extrakorporalen Kreislauf 4 zu dem Dialysierflüssigkeitskreislauf 8 sowohl über Diffusion als auch über Konvektion erfolgt, enthält der Beutel 48 Dialysierflüssigkeit. Wird nun ein erstes Ventil 50 geöffnet und werden sowohl ein zweites Ventil 52 als auch ein drittes Ventil 54 geschlossen, kann die Dialysierflüssigkeit über eine Pumpe 56 zum Dialysator 6 gepumpt werden.

Soll alternativ mit der extrakorporalen Blutbehandlungsvorrichtung 2 beispielsweise eine Hämofiltration etc. durchgeführt werden, also eine Blutbehandlungstherapie, in welcher keine Dialysierflüssigkeit durch den Dialysator 6 strömt und somit ein Stofftransport von dem extrakorporalen Kreislauf 4 zu dem Dialysierflüssigkeitskreislauf 8 lediglich über Konvektion/ Filtration erfolgt, kann der Beutel 48 eine Substitutionslösung enthalten. Werden nun das erste Ventil 50 und das zweite Ventil 52 geschlossen und wird das dritte Ventil 54 geöffnet, kann die Substitutionslösung aus dem Beuel 48 in den arteriellen Abschnitt 12 des extrakorporalen Kreislaufs 4 gepumpt werden (Prädilution). Werden das erste Ventil 50 und das dritte Ventil 54 geschlossen und wird das zweite Ventil 52 geöffnet, kann die Substitutionslösung aus dem Beuel 48 in den venösen Abschnitt 14 des extrakorporalen Kreislaufs 4 gepumpt werden (Postdilution). Wird das erste Ventil 50 geschlossen und werden das zweite Ventil 52 und das dritte Ventil 54 geöffnet, kann die Substitutionslösung aus dem Beutel 48 sowohl in den arteriellen Abschnitt 12 als auch in den venösen Abschnitt 14 des extrakorporalen Kreislaufs gepumpt werden (Prä- und Postdilution). Eine Prä- und Postdilution kann gemäß der vorliegenden Offenbarung auch dadurch erreicht werden, dass die Substitutionslösung aus dem Substitutionslösungsbeutel 34 mittels der Substitutionslösungspumpe 36 in den venösen Abschnitt 14 des extrakorporalen Kreislaufs 4 gepumpt wird (Postdilution), und dass gleichzeitig die Substitutionslösung aus dem Beutel 48 mittels der Pumpe (Substitutionslösungspumpe) 56 in den arteriellen Abschnitt 12 des extrakorporalen Kreislaufs 4 gepumpt wird (Prädilution).

Wie aus Fig. 1 weiter hervorgeht, sind zwischen der Pumpe 56 und der Ventilanordnung bestehend aus dem ersten Ventil 50, dem zweiten Ventil 52 und dem dritten Ventil 54 ein Flüssigkeitswärmer 58 und ein Drucksensor 60 vorgesehen.

An den drei Beuteln, also dem Substitutionslösungsbeutel 34, dem Sammelbeutel 40 und dem Beutel 48, sind jeweils Wägezellen, nämlich eine erste Wägezelle 62, eine zweite Wägezelle 64 und eine dritte Wägezelle 66 angeordnet. Die erste Wägezelle 62 ist dabei grundsätzlich eingerichtet, das Gewicht des Substitutionslösungsbeutels 34 zu messen bzw. zu überwachen. Die zweite Wägezelle 64 ist grundsätzlich eingerichtet, das Gewicht des Sammelbeutels 40 zu messen bzw. zu überwachen. Die dritte Wägezelle 66 ist grundsätzlich eingerichtet, das Gewicht des Beutels 48 zu messen bzw. zu überwachen.

Die extrakorporale Blutbehandlungsvorrichtung 2 weist weiterhin eine Steuereinheit (CPU) 68 auf, welche Informationen von den in der Blutbehandlungsvorrichtung 2 vorgesehenen Sensoren erhält, und welche die in der Blutbehandlungsvorrichtung 2 vorgesehenen Aktoren ansteuert. Offenbarungsgemäß wird somit insbesondere eine softwareunterstützte Therapie bereitgestellt. Die Steuereinheit 68 erhält dabei insbesondere Informationen von dem arteriellen Drucksensor 18, dem Dialysatoreingangsdrucksensor 22, dem Sicherheitsluftdetektor 28, dem venösen Drucksensor 32, dem Ultrafiltratdrucksensor 42, dem Blutleckdetektor 44, dem Drucksensor 60, der ersten Wägezelle 62, der zweiten Wägezelle 64, der dritten Wägezelle 66, etc. Die Steuereinheit 68 steuert insbesondere die Blutpumpe 20, das Sicherheitsventil 30, die Substitutionslösungspumpe 36, die Ultrafiltratpumpe 46, das erste Ventil 50, das zweite Ventil 52, das dritte Ventil 54, die Pumpe 56, den Flüssigkeitswärmer 58, etc. an. Weiterhin ist die Steuereinheit 68 im Austausch mit einer als Display mit Touch-Screen ausgebildeten Benutzeroberfläche 70. Beispielsweise kann die Steuereinheit 68 eingerichtet sein, eine Warnung oder einen Alarm auf der Benutzeroberfläche 70 anzuzeigen. Weiterhin können von einem Nutzer/ Anwender auf der Benutzeroberfläche 70 eingegebene Informationen an die Steuereinheit 68 weitergegeben werden.

Wie aus Fig. 1 bereits hervorgeht, betrifft die vorliegende Offenbarung im Wesentlichen die Ansteuerung der Substitutionslösungspumpe 36 und der Pumpe 56 (wenn die Pumpe 56 als Substitutionslösungspumpe arbeitet). Die vorliegende Offenbarung betrifft insgesamt im Wesentlichen eine durch die Steuereinheit 68 vorgenommene Steuerung. Die Steuereinheit 68 kann insbesondere eine Differenz bzw. einen Rückstand zwischen einem idealen/ optimalen, durch einen Anwender eingestellten Sollvolumen der zugeführten Substitutionslösung und einem tatsächlich gesteuerten Volumen der zugeführten Substitutionslösung berechnen. Dazu verwendet die Steuereinheit 68 einen zeitlichen Verlauf der Flussrate der Substitutionslösungspumpe 36 oder der Pumpe 56.

Wenn die Steuereinheit 68 feststellt/ wenn der Steuereinheit 68 bekannt wird (durch eine entsprechende Berechnung), dass eine Differenz bzw. ein Rückstand zwischen einem idealen/ optimalen, durch einen Anwender eingestellten Sollvolumen und einem tatsächlich/ wirklich gesteuerten Volumen der zugeführten Substitutionslösung vorliegt, erhöht die Steuereinheit 68 temporär eine gesteuerte Flussrate der Substitutionslösungspumpe 36 oder der Pumpe 56 um einen vorbestimmten festen Prozentsatz. Das bedeutet, dass die Flussrate der Substitutionslösungspumpe 36 oder der Pumpe 56 um einen vorbestimmten festen Prozentsatz größer als eine normalerweise erforderliche Flussrate eingestellt wird. Unter einer normalerweise erforderlichen Flussrate ist eine Flussrate zu verstehen, mittels welcher das ideale/ optimale, durch einen Anwender eingestellte Sollvolumen erreichbar wäre, wenn kein Rückstand/ keine Differenz zwischen dem eingestellten Sollvolumen und dem tatsächlich gesteuerten Volumen der zugeführten Substitutionslösung vorliegen würde.

Der vorbestimmte feste Prozentsatz kann grundsätzlich auf einen Wert zwischen 1% und 5% eingestellt sein. Es kann auch vorgesehen sein, dass der vorbestimmte, feste Prozentsatz höher eingestellt wird, wenn die Abweichung zwischen dem tatsächlichen Volumen und dem Sollvolumen groß ist als wenn die Abweichung zwischen dem tatsächlichen Volumen und dem Sollvolumen klein ist. Etwa kann der vorbestimmte feste Prozentsatz auf 1% eingestellt werden, wenn die Abweichung klein ist, und kann der vorbestimmte feste Prozentsatz auf 5% eingestellt werden, wenn die Abweichung groß ist. In jedem Fall ist jedoch der Prozentsatz, welcher von der Steuereinheit eingestellt wird, (in Abhängigkeit von der Differenz/ dem Rückstand) bereits voreingestellt und vorbestimmt.

Offenbarungemäß wird die Flussrate/ der Volumenstrom der Substitutionslösungspumpe 36 oder der Pumpe 56 so lange um den vorbestimmten festen Prozentsatz erhöht, bis die Differenz bzw. der Rückstand zwischen dem tatsächlich gesteuerten Volumen und dem idealen Sollvolumen nicht mehr vorliegt, also das tatsächlich gesteuerte Volumen (wieder) dem idealen Sollvolumen entspricht.

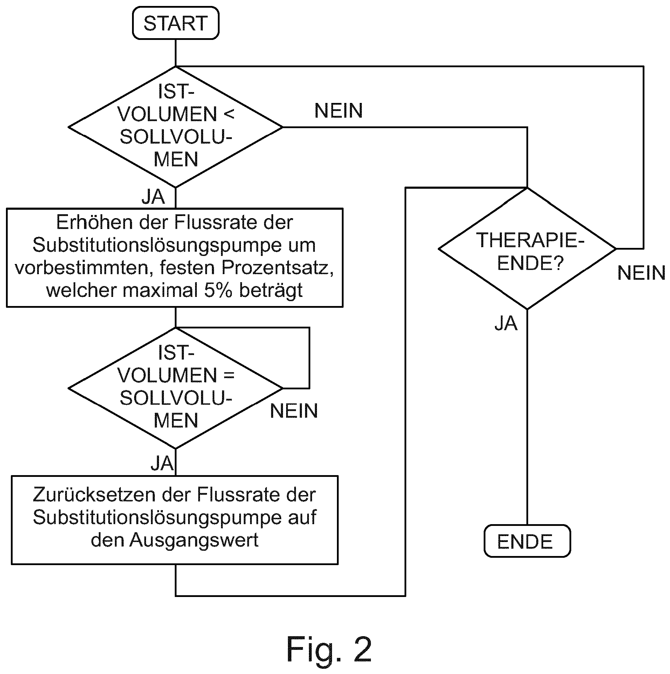

Fig. 2 zeigt den offenbarungsgemäßen Ablauf einer automatischen Volumenkompensation einer Substitutionslösung. Die Steuereinheit 68 berechnet zunächst ein tatsächlich gesteuertes Volumen der Substitutionslösung, welches einem extrakorporalen Kreislauf 4 zugeführt wird. Dann vergleicht die Steuereinheit 68 das tatsächlich gesteuerte, dem extrakorporalen Kreislauf 4 zugeführte Volumen mit einem (vorbestimmten) idealen Sollvolumen. Wenn das tatsächlich zugeführte Volumen bzw. Istvolumen kleiner als das ideale Sollvolumen ist, erhöht die Steuereinheit die Flussrate einer Substitutionslösungspumpe um einen vorbestimmten, festen Prozentsatz, welcher maximal 5% beträgt. Dann vergleicht die Steuereinheit 68 weiterhin das Sollvolumen mit dem Istvolumen. Erst wenn das Sollvolumen gleich dem Istvolumen ist, setzt die Steuereinheit 68 die Flussrate der Substitutionslösungspumpe auf den Ausgangswert/ eigentlich erforderlichen Wert zurück. Die dargestellte Routine endet erst, wenn ein Therapieende vorliegt.

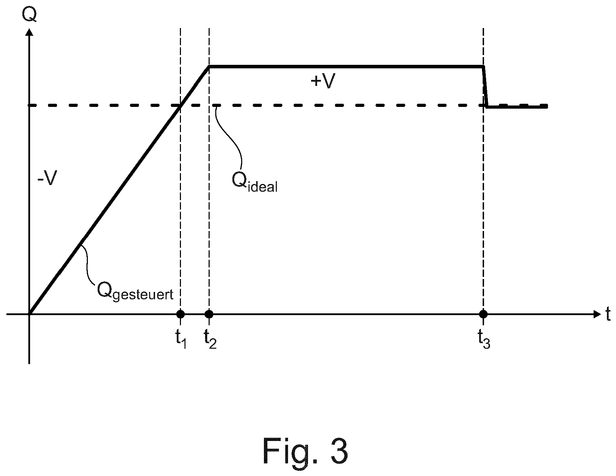

Fig. 3 zeigt ein Diagramm, in welchem ein zeitlicher Verlauf einer von der Steuereinheit 68 gesteuerten Substitutionslösungsflussrate Q_{gesteuert} der Substitutionslösungspumpe 36 oder der Pumpe 56 dargestellt ist. Insbesondere zeigt Fig. 3, dass sich bei einem Starten oder einem erneuten Starten der Substitutionslösungspumpe 36 oder der Pumpe 56 die Substitutionslösungsflussrate Q_{gesteuert} langsam/ kontinuierlich/ linear (von null) erhöht, so dass eine erwünschte, von einem Anwender eingestellte ideale Flussrate Q_{ideal}, welche zur Folge hätte, dass das ideale/ optimale Sollvolumen dem extrakorporalen Kreislauf 4 zugeführt werden würde (wenn es bereits von Beginn an eingestellt wäre/ vorliegen würde), erst zu einem Zeitpunkt t1 erreicht wird. Gemäß der vorliegenden Offenbarung wird die gesteuerte Substitutionslösungsflussrate Q_{gesteuert} ab Zeitpunkt t1 (noch) nicht auf die von dem Anwender eingestellt ideale Flussrate Q_{ideal} eingestellt, sondern steigt zunächst weiter linear an, und zwar bis eine gesteuerte Flussrate Q_{gesteuert} erreicht wird, welche gegenüber der idealen Flussrate Q_{ideal} um einen vorbestimmten, festen Prozentsatz erhöht ist. Dies ist in Fig. 3 bei Zeitpunkt t2 der Fall. Nun wird die gesteuerte Flussrate temporär bei einem konstanten Wert gehalten, und zwar solange bis das beim Starten noch nicht zugeführte Volumen (siehe "-V" in Fig. 3), das heißt der Rückstand bzw. die Differenz, vollständig kompensiert wurde (siehe "+V in Fig. 3). Dies ist in Fig. 3 bei Zeitpunkt t3 der Fall. Zu Zeitpunkt t3 wird die gesteuerte Flussrate Q_{gesteuert} schließlich auf die ideale Flussrate Q_{ideal} eingestellt.

### Bezugszeichenliste

- 2: Blutbehandlungsvorrichtung/ Dialysemaschine
- 4: extrakorporaler Kreislauf
- 6: Dialysator
- 8: Dialysierflüssigkeitskreislauf
- 10: Membran
- 12: arterieller Abschnitt
- 14: venöser Abschnitt
- 16: arterielles Ende
- 18: arterieller Drucksensor
- 20: (arterielle) Blutpumpe
- 22: Dialysatoreingangsdrucksensor
- 24: venöses Ende
- 26: venöse Expansionskammer/ Luftfalle
- 28: Sicherheitsluftdetektor
- 30: Sicherheitsventil
- 32: venöser Drucksensor
- 34: Substitutionslösungsbeutel
- 36: Substitutionslösungspumpe
- 38: Abfluss
- 40: Sammelbeutel
- 42: Ultrafiltratdrucksensor
- 44: Blutleckdetektor
- 46: Ultrafiltratpumpe
- 48: Beutel
- 50: erstes Ventil
- 52: zweites Ventil
- 54: drittes Ventil
- 56: Pumpe
- 58: Flüssigkeitswärmer
- 60: Drucksensor
- 62: erste Wägezelle
- 64: zweite Wägezelle
- 66: dritte Wägezelle
- 68: Steuereinheit
- 70: Benutzerschnittstelle

## Patentansprüche

1. Blutbehandlungsvorrichtung (2), insbesondere Dialysemaschine, zur Verwendung in Blutbehandlungstherapien, insbesondere Nierenersatztherapien, mit:
einem extrakorporalen Blutkreislauf (4), einem Dialysator (6) und einem Dialysierflüssigkeitskreislauf (8), wobei der extrakorporale Blutkreislauf (4) und der Dialysierflüssigkeitskreislauf (8) über eine in dem Dialysator (6) vorgesehene Membran (10), über welche Blut gefiltert werden kann, voneinander getrennt sind;
zumindest einer Substitutionslösungspumpe (36, 56), welche eingerichtet ist, eine Substitutionslösung dem extrakorporalen Blutkreislauf (4) vor und/oder nach dem Dialysator (6) zuzuführen;
**gekennzeichnet durch**
einer Steuereinheit (68), welche eingerichtet ist, einen Rückstand zwischen einem idealen, durch einen Anwender eingestellten Sollvolumen und einem tatsächlich gesteuerten Volumen der zugeführten Substitutionslösung zu berechnen, und eine gesteuerte Flussrate der Substitutionslösungspumpe (36, 56) temporär unter entsprechender Ansteuerung derselben um einen vorbestimmten festen Prozentsatz, welcher kleiner oder gleich 5 % ist, zu erhöhen, und zwar so lange, bis der Rückstand zwischen dem tatsächlich gesteuerten Volumen und dem idealen Sollvolumen nicht mehr vorliegt, also das tatsächlich gesteuerte Volumen dem idealen Sollvolumen entspricht.

2. Blutbehandlungsvorrichtung (2) nach Anspruch 1, **dadurch gekennzeichnet, dass** der vorbestimmte, feste Prozentsatz, um welchen die Flussrate der Substitutionslösung erhöht wird, zumindest 1% und höchstens 5% ist.

3. Blutbehandlungsvorrichtung (2) nach Anspruch 1 oder 2, **dadurch gekennzeichnet, dass** der vorbestimmte, feste Prozentsatz von der Steuereinheit (68) in Abhängigkeit von dem fehlenden Volumen eingestellt wird, so dass der vorbestimmte, feste Prozentsatz höher eingestellt wird, wenn die Abweichung zwischen dem tatsächlichen Volumen und dem Sollvolumen groß ist als wenn die Abweichung zwischen dem tatsächlichen Volumen und dem Sollvolumen klein ist.

4. Blutbehandlungsvorrichtung (2) nach einem der vorhergehenden Ansprüche, **dadurch gekennzeichnet, dass** die Steuereinheit (68) eingerichtet ist, die Flussrate der Substitutionslösungspumpe (36, 56) nur dann zu erhöhen, wenn dies nicht andere Restriktionen verbieten.

5. Blutbehandlungsvorrichtung (2) nach einem der vorhergehenden Ansprüche, **dadurch gekennzeichnet, dass** die Steuereinheit (68) eingerichtet ist, den Rückstand zwischen dem idealen Sollvolumen und dem tatsächlich gesteuerten Volumen unter Verwendung des Verlaufs der Flussrate der zumindest einen Substitutionslösungspumpe (36, 56) zu berechnen.

6. Blutbehandlungsvorrichtung (2) nach einem der vorhergehenden Ansprüche, **dadurch gekennzeichnet, dass** die Steuereinheit (68) eingerichtet ist, die Flussrate der zumindest einen Substitutionslösungspumpe (36, 56) einzustellen.

7. Blutbehandlungsvorrichtung (2) nach Anspruch 6, **dadurch gekennzeichnet, dass** sich beim Starten oder erneuten Starten der zumindest einen Substitutionslösungspumpe (36, 56) die Flussrate langsam erhöht, so dass eine erwünschte ideale Flussrate erst nach einer vorbestimmten kurzen Zeitspanne erreicht wird.

8. Blutbehandlungsvorrichtung (2) nach Anspruch 7, **dadurch gekennzeichnet, dass** die Flussrate nach einem Erreichen der erwünschten idealen Flussrate temporär um den vorbestimmten festen Prozentsatz erhöht wird, um den Rückstand zwischen dem tatsächlich gesteuerten Volumen und dem idealen Sollvolumen, welcher von der langsamen Erhöhung der Flussrate beim Starten oder erneuten Starten resultiert, langsam zu verringern, und zwar so lange, bis der Rückstand zwischen dem tatsächlich gesteuerten Volumen und dem idealen Sollvolumen nicht mehr vorliegt, also das tatsächlich gesteuerte Volumen dem idealen Sollvolumen entspricht.

9. Blutbehandlungsvorrichtung (2) nach einem der vorhergehenden Ansprüche, **dadurch gekennzeichnet, dass** die Steuereinheit (68) eingerichtet ist, wenn die gesteuerte Flussrate der Substitutionslösungspumpe (36, 56) temporär reduziert werden muss, den daraus resultierenden Rückstand zwischen dem idealen Sollvolumen und dem tatsächlich gesteuerten Volumen im Nachhinein wieder zu verringern bzw. auszugleichen, indem die gesteuerte Flussrate der Substitutionslösungspumpe (36, 56) temporär unter entsprechender Ansteuerung derselben um den vorbestimmten festen Prozentsatz erhöht wird, und zwar so lange, bis der Rückstand zwischen dem tatsächlich gesteuerten Volumen und dem idealen Sollvolumen nicht mehr vorliegt, also das tatsächlich gesteuerte Volumen dem idealen Sollvolumen entspricht.

## Claims

1. Blood treatment device (2), in particular dialysis device, for use in blood treatment therapies, in particular renal replacement therapies, comprising:
an extracorporeal blood circuit (4), a dialyzer (6) and a dialysis fluid circuit (8), wherein the extracorporeal blood circuit (4) and the dialysis fluid circuit (8) are separated from each other via a membrane (10) provided in the dialyzer (6), via which blood can be filtered;
at least one substitution solution pump (36, 56), which is configured to supply a substitution solution to the extracorporeal blood circuit (4) before and/or after the dialyzer (6);
**characterized by**
a control unit (68) which is configured to calculate a backlog between an ideal target volume set by a user and an actually controlled volume of the supplied substitution solution, and to temporarily increase a controlled flow rate of the substitution solution pump (36, 56) under corresponding driving thereof by a predetermined, fixed percentage which is less than or equal to 5%, specifically until the backlog between the actually controlled volume and the ideal target volume no longer exists, i.e. the actually controlled volume corresponds to the ideal target volume.

2. Blood treatment device (2) according to claim 1, **characterized in that** the predetermined, fixed percentage by which the flow rate of the substitution solution is increased is at least 1% and at most 5%.

3. Blood treatment device (2) according to claim 1 or 2, **characterized in that** the predetermined, fixed percentage is set by the control unit (68) depending on the missing volume, so that the predetermined, fixed percentage is set higher when the deviation between the actual volume and the target volume is large, than when the deviation between the actual volume and the target volume is small.

4. Blood treatment device (2) according to one of the preceding claims, **characterized in that** the control unit (68) is configured to increase the flow rate of the substitution solution pump (36, 56) only if other restrictions do not prohibit this.

5. Blood treatment device (2) according to one of the preceding claims, **characterized in that** the control unit (68) is configured to calculate the backlog between the ideal target volume and the actually controlled volume using the course of the flow rate of the at least one substitution solution pump (36, 56).

6. Blood treatment device (2) according to one of the preceding claims, **characterized in that** the control unit (68) is configured to adjust the flow rate of the at least one substitution solution pump (36, 56).

7. Blood treatment device (2) according to claim 6, **characterized in that** when starting or restarting the at least one substitution solution pump (36, 56), the flow rate slowly increases so that a desired ideal flow rate is reached only after a predetermined, short time period.

8. Blood treatment device (2) according to claim 7, **characterized in that** after reaching the desired ideal flow rate, the flow rate is temporarily increased by the predetermined, fixed percentage in order to slowly reduce the backlog between the actually controlled volume and the ideal target volume, which results from the slow increase of the flow rate at start-up or restart, specifically until the backlog between the actually controlled volume and the ideal target volume no longer exists, i.e. the actually controlled volume corresponds to the ideal target volume.

9. Blood treatment device (2) according to one of the preceding claims, **characterized in that** the control unit (68) is configured so that, if the controlled flow rate of the substitution solution pump (36, 56) has to be temporarily reduced, the resulting backlog between the ideal target volume and the actually controlled volume is subsequently reduced or compensated for, by temporarily increasing the controlled flow rate of the substitution solution pump (36, 56) by the predetermined, fixed percentage under appropriate driving thereof, specifically until the backlog between the actually controlled volume and the ideal target volume no longer exists, i.e. the actually controlled volume corresponds to the ideal target volume.

## Revendications

1. Dispositif de traitement du sang (2), en particulier machine de dialyse, pour l'utilisation dans des thérapies de traitement du sang, en particulier des thérapies de substitution du rein, avec :
un circuit extracorporel du sang (4), un dialyseur (6) et un circuit de liquide de dialyse (8), dans lequel le circuit extracorporel du sang (4) et le circuit de liquide de dialyse (8) sont séparés l'un de l'autre par le biais d'une membrane (10) prévue dans le dialyseur (6), par le biais de laquelle du sang peut être filtré ;
au moins une pompe de solution de substitution (36, 56) qui est conçue afin d'amener une solution de substitution au circuit extracorporel du sang (4) avant et/ou après le dialyseur (6) ;
**caractérisé par**
un dispositif de commande (68) qui est conçu afin de calculer un résidu entre un volume de consigne idéal réglé par un utilisateur et un volume commandé réellement de la solution de substitution amenée, et d'augmenter temporairement un débit commandé de la pompe de solution de substitution (36, 56) en commandant de manière correspondante celui-ci d'un taux de pourcentage fixe prédéterminé qui est inférieur ou égal à 5 %, et ce tant que le résidu entre le volume commandé réellement et le volume de consigne idéal n'existe plus, donc le volume commandé réellement correspond au volume de consigne idéal.

2. Dispositif de traitement du sang (2) selon la revendication 1, **caractérisé en ce que** le taux de pourcentage fixe prédéterminé, duquel le débit de la solution de substitution est augmenté, est au moins de 1 % et au plus de 5 %.

3. Dispositif de traitement du sang (2) selon la revendication 1 ou 2, **caractérisé en ce que** le taux de pourcentage fixe prédéterminé est réglé par le dispositif de commande (68) en fonction du volume manquant de sorte que le taux de pourcentage fixe prédéterminé soit réglé plus haut lorsque l'écart entre le volume réel et le volume de consigne est grand que lorsque l'écart entre le volume réel et le volume de consigne est petit.

4. Dispositif de traitement du sang (2) selon l'une quelconque des revendications précédentes, **caractérisé en ce que** le dispositif de commande (68) est conçu afin d'augmenter le débit de la pompe de solution de substitution (36, 56) seulement lorsque cela n'empêche pas d'autre restrictions.

5. Dispositif de traitement du sang (2) selon l'une quelconque des revendications précédentes, **caractérisé en ce que** le dispositif de commande (68) est conçu afin de calculer le résidu entre le volume de consigne idéal et le volume commandé réellement en utilisant l'étendue du débit de l'au moins une pompe de solution de substitution (36, 56).

6. Dispositif de traitement du sang (2) selon l'une quelconque des revendications précédentes, **caractérisé en ce que** le dispositif de commande (68) est conçu afin de régler le débit de l'au moins une pompe de solution de substitution (36, 56).

7. Dispositif de traitement du sang (2) selon la revendication 6, **caractérisé en ce que** lors du démarrage ou redémarrage de l'au moins une pompe de solution de substitution (36, 56) le débit augmente lentement de sorte qu'un débit idéal souhaité ne soit atteint qu'après un bref intervalle prédéterminé.

8. Dispositif de traitement du sang (2) selon la revendication 7, **caractérisé en ce que** le débit après une atteinte du débit idéal souhaité est augmenté temporairement du taux de pourcentage fixe prédéterminé afin de réduire lentement le résidu entre le volume commandé réellement et le volume de consigne idéal qui résulte de l'augmentation lente du débit lors du démarrage ou redémarrage, et ce tant que le résidu entre le volume commandé réellement et le volume de consigne idéal n'existe plus, donc le volume commandé réellement correspond au volume de consigne idéal.

9. Dispositif de traitement du sang (2) selon l'une quelconque des revendications précédentes, **caractérisé en ce que** le dispositif de commande (68) est conçu lorsque le débit commandé de la pompe de solution de substitution (36, 56) doit être réduit temporairement afin de réduire à nouveau ou de compenser le résidu en résultant entre le volume de consigne idéal et le volume commandé réellement a posteriori, **en ce que** le débit commandé de la pompe de solution de substitution (36, 56) est augmenté temporairement en commandant de manière correspondante celui-ci du taux de pourcentage fixe prédéterminé, et ce tant que le résidu entre le volume commandé réellement et le volume de consigne idéal n'existe plus, donc le volume commandé réellement correspond au volume de consigne idéal.
